**Europäisches Patentamt**

**European Patent Office**

**Office européen des brevets**

(11) Publication number: **0 060 451**
**B1**

# EUROPEAN PATENT SPECIFICATION

(45) Date of publication of patent specification: **17.09.86**

(51) Int. Cl.⁴: **A 61 N 1/30, A 61 N 1/04**

(21) Application number: **82101692.0**

(22) Date of filing: **04.03.82**

(54) Iontophoretic electrode.

(30) Priority: **06.03.81 US 241150**

(43) Date of publication of application:
**22.09.82 Bulletin 82/38**

(45) Publication of the grant of the patent:
**17.09.86 Bulletin 86/38**

(84) Designated Contracting States:
**CH DE FR GB IT LI NL SE**

(56) References cited:
**EP-A-0 029 297**
**DE-A-2 724 461**
**DE-A-2 740 270**
**GB-A- 410 009**
**US-A-3 315 665**
**US-A-3 716 054**
**US-A-3 794 910**
**US-A-4 002 221**
**US-A-4 248 247**
**US-A-4 273 135**

(73) Proprietor: **MEDTRONIC, INC.**
**3055 Old Highway Eight**
**Minneapolis Minnesota 55440 (US)**

(72) Inventor: **Spevak, Richard P.**
**2119 Ardan Ave.**
**Mounds View, MN 55432 (US)**
Inventor: **Lattin, Gary A.**
**6823 145th Avenue NE**
**Forest Lake, MN 55025 (US)**
Inventor: **Jevne, Allan H.**
**1314 153 rd LaneNE**
**Anoka, MN 55303 (US)**

(74) Representative: **Schwan, Gerhard, Dipl.-Ing.**
**Elfenstrasse 32**
**D-8000 München 83 (DE)**

Courier Press, Leamington Spa, England.

## Description

This invention relates to a iontophoretic device for the iontophoretic introduction of a ionic drug into body tissue.

Iontophoresis is a method for introducing ionic substances into a body. The method utilizes direct electrical current to drive the ionized substances, such as chemicals or drugs, through the intact skin or other body surface. This has proven to be very useful in numerous medical applications (US—A—3 991 755 and US—A—4 141 359). The iontophoresis process has been found to be useful in the administration of lidocaine hydrochloride, hydrocortisone derivatives, acetic acid, fluoride, penicillin, dexamethasone sodium phosphate and many other drugs. Perhaps the widest use of iontophoresis is that of diagnosing cystic fibrosis by using pilocarpine nitrate iontophoresis. The pilocarpine nitrate stimulates sweat production; the sweat is collected and analyzed for its chloride or sodium content to detect the presence of the disease.

In iontophoretic devices two electrodes are used. One electrode, called the active electrode, is the electrode at which the ionic substance is driven into the body. The other electrode, called the indifferent or ground electrode, serves to close the electrical circuit through the body. The active electrode must hold, contain, or otherwise have available to it a source of the ionic substance. Thus, in the prior art the active electrode is generally relatively complex compared to the indifferent electrode.

US—A—3 991 755 discloses iontophoretic devices having several examples of active electrodes. In one example the electrode comprises a stainless steel wire housed in a plastic sheath which is shaped to safely fit within the ear canal together with a liquid solution containing the ionized substance which liquid is poured into the ear canal so that it contacts the inner ear and the wire through an opening in the sheath. In another example, the electrode wire housed in a plastic sheath having an opening is placed in a wad of absorbent material which holds the liquid containing the ionic substance.

US—A—4 141 359 also discloses several embodiments of iontophoretic devices comprising an active electrode. All of the embodiments include a receptacle for holding either a conducting gel in which the ionic substance is dissolved, or for holding a sponge which is saturated with the conductive gel in which the ionic substance is dissolved. The conducting gel/ionic drug solution communicates with the body tissue through a hole in the receptacle. The receptacle is held in contact with the skin by an adhesive pad surrounding the receptacle or a strap attached to the pad. In other embodiments of the prior art the hole in the receptacle is covered with a membrane and the ionic substance is driven through the membrane by the electric current.

An active electrode formed by a gauze pad soaked in the solution containing the ionic substance superimposed by several layers of paper towels moistened with tap water and a section of block tin or aluminum foil placed over the moistened towel, with the tin or foil connected to the iontophoretic current generator by means of a wire and alligator clip is disclosed in *Acetic Acid Iontophoresis for Calcium Deposits*, by Joseph Kahn in *Physical Therapy*, Vol. 57, No. 6, June 1977 (pp. 658—659).

GB—A—410 009 discloses an apparatus for iontophoretic introduction of medicaments into the human or animal body, comprising a galvanic cell having two electrodes made of materials possessing different electrical potentials, and a layer of material, such as a piece of filtering paper, gauze or the like, impregnated with the medicament, with an electrolyte and with an adhesive substance, in direct contact with the anode.

The aforementioned prior devices have a number of disadvantages. Those in which the ionic substance of the active electrode is held in solution in a liquid are relatively messy. Of these, those in which the liquid is not contained in an absorbent material can be used only in situations where body cavities form a natural container for the liquid, or it is necessary to employ a cup or other container to hold the liquid about the body surface through which the ionic substance can be driven. The devices having electrodes employing the conductive gel in which the ionic substance is dissolved are somewhat less messy, but still leave a residue of gel after use. Devices the electrodes of which employ the membrane alleviate most of the above problems but create additional complexities in the construction and handling of the electrode, relating both to the membrane itself and the separate means for insertion of the ionic substance that is required if a membrane is used. In all of these devices, a separate strap or adhesive pad is necessary to hold the electrode in place, or alternatively the patient must remain still during the use of the iontophoretic device so that the electrode will remain in place. Even with the use of a strap or adhesive pad the portion of the electrode containing the ionic substance is still subject to some movement due to the flexibility in such materials, and the flexibility of body tissue. Moreover, in all of these devices some of the liquid or gel may move away from the main body of the liquid gel, as for example by dripping out of the absorbent material or sliding under the edge of the adhesive pad. When this occurs, electrical contact may or may not be lost and the ionic substance in the dislocated portion of the material may or may not be driven into the body. The aforementioned disadvantages all result in an inability to precisely control the area over which the ionic substance is administered, which control is often necessary for drugs for which the device is used. Furthermore the active electrodes of the aforementioned devices require complex procedures for handling the material containing the ionic substance prior to and, in some electrodes, during the iontophoretic process.

EP—A—0 029 297 (prior art in conformity with Article 54 (3) EPC) discloses an iontophoresis electrode of the type having an electrically conductive, non-liquid, self-adhesive, disposable skin interfacing member and a non-disposable backer member from which the skin interfacing member may peeled away. The interfacing member may be a flat cohesive sheet of adhesive and electrically conductive material. An electrically conductive snap connector is secured through the backer member to make physical contact with the interfacing member. An electrical lead is adapted to be connected to the snap connector.

A simpler device would permit the iontophoretic process to be much more widely applied, not only in applications now known to be practiced, but also in many new applications that previously were not practical. For example, although electrical stimulation of body tissue for pain suppression and muscular therapy have been known for some time (see, for example, U.S. Patent No. 4,019,518) these treatments have not, up to now, been used in combination with iontophoresis, except on a limited clinical basis, since up to now iontophoretic devices have been too messy and complex for use by the average patient.

The object basic to the invention is to provide a iontophoretic device for iontophoretically introducing an ionic drug into body tissue, which device eliminates the need for additional parts to contain and/or separate the ionic drug.

In conformity with the invention this object is reached by an iontophoretic device for iontophoretically introducing an ionic drug into body tissue, through the skin, said device comprising a housing for a pulse generator having affixed thereto at least one electrode plate which has removably applied thereto an adhesive iontophoretic electrode sheet composed of a mixture of an adhesive and said ionic drug, one of the broad surfaces of said adhesive sheet electrically coupling the electrode sheet to the electrode plate and the other of the broad surfaces of said adhesive sheet serving as means for coupling the adhesive electrode sheet and thereby the housing to the skin.

In an iontophoretic electrode sheet for use in such a device preferably the adhesive is a non-ionic, polar adhesive. Other substances may be included in the electrode sheet so long as they do not materially and adversely affect the iontophoretic process and the adhesive quality of the mixture. In the present device the electrode sheet serves as the container for the ionic drug, the means for attaching the electrode to the body, the means for electrically coupling the electrode to the electrode plate, the means for conducting the ionic substance into the body, and also as a means for attaching the iontophoretic device to the body.

Numerous advantages arise from the combination of the ionic substance with the adhesive in the electrode. In the present device the electrode sheet serves as a means for attaching the electrode to the body, and thus straps, adhesive attachments pads, etc. may be simplified or eliminated in some embodiments. The present device also provides for precise delivery of dosages of the ionic substance, since the electrode composition tends to cling together and portions of it cannot easily become separated. Moreover, the entire active surface of the electrode is held firmly to the body, even under the condition of considerable movement of the body, which again serves to facilitate precise delivery, and also serves to facilitate uniform dosage control over the body portion to which the electrode is applied. The electrode sheets can be made very simple since the electrode sheet serves as its own receptacle. Thus receptacle structures such as a cup, absorbent swab, membranes, etc. are eliminated.

In using the device of the invention an electrode containing a mixture of the ionic substance and an adhesive material may be applied to the electrode plate, and the device may be applied to the body. Then an electric current may be driven through the mixture and into the body thereby introducing the ionic substance into the body. The device may constitute a combination iontophoretic/electrical pulse stimulator, i.e. an iontophoretic generator that also includes an electrical nerve stimulator, an electrical muscle stimulator or a combination of both types of stimulation.

The device according to the invention advantageously may be used in the diagnosis of cystic fibrosis wherein the electrode contains a mixture of pilocarpine nitrate in an adhesive material. Upon application of the device to the body, an electric current is driven through the electrode and into the body to drive the pilocarpine nitrate into the body, the body is allowed to produce sweat, and the sweat is analyzed to determine whether cystic fibrosis is present.

The present device facilitates the mass application of such a diagnostic process, for example in the mass screening of newborn infants for cystic fibrosis.

Numerous other features, objects and advantages of the invention will become apparent from the following detailed description when read in conjunction with the accompanying drawing.

In the drawing:

Fig. 1 is a schematic circuit diagram of an exemplary iontophoretic device;

Fig. 2 is a sectional diagrammatic view of a pair of electrodes of a iontophoretic device applied to the body, illustrating the iontophoretic process;

Fig. 3 is a sectional view of a preferred embodiment of an active electrode of the iontophoretic device of the invention;

Fig. 4 is a side view of a iontophoretic device according to the invention employing an alternative preferred embodiment of an electrode with one electrode in place;

Fig. 5a is a side view showing the electrode that is employed with the device of Fig. 4, in the form in which the electrode may be manufactured and sold;

Fig. 5b is a side view of the electrode of Fig. 5a showing one of the protective covering sheets removed, in preparation for the application of the electrode to the device of Fig. 4; and

Fig. 6 is a bottom view of the iontophoretic device of Fig. 4.

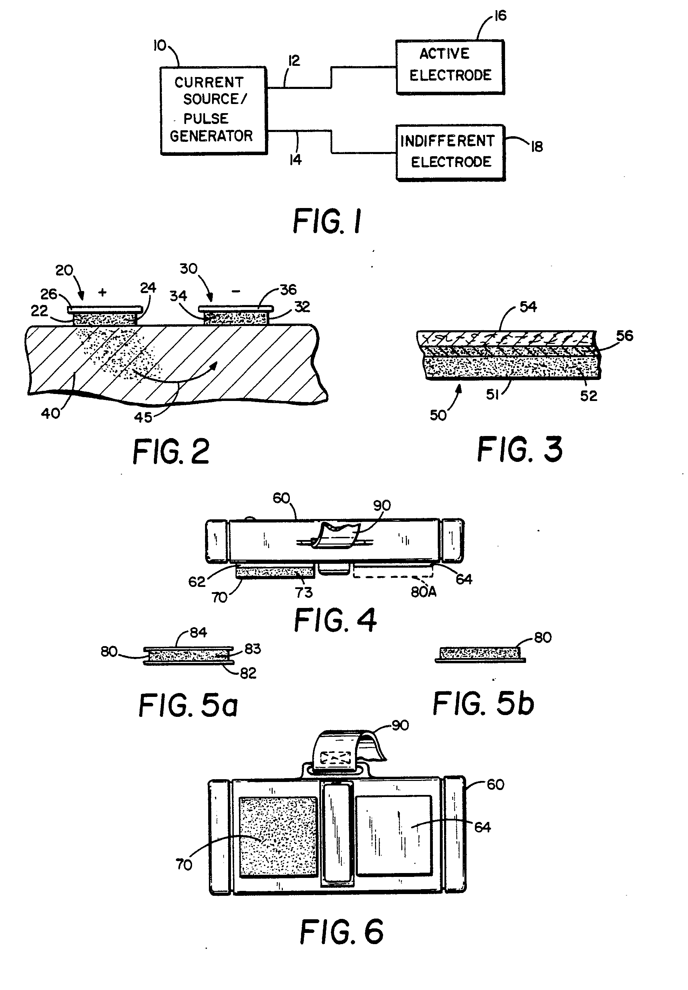

As illustrated in diagrammatic form in Fig. 1 the iontophoretic device includes current source 10 which is electrically coupled through electrically conductive members 12 and 14 to electrodes 16 and 18, respectively. For purposes of illustration, electrode 16 is labeled the "active" electrode while electrode 18 is labeled the "indifferent" electrode, although the positions may be reversed. It is also possible in some embodiments that an electrode that is active when the current is flowing in one direction in the system will become an indifferent electrode when the current is flowing the opposite direction in the system.

Fig. 2 shows a sectional view of a pair of electrodes 20 and 30, of a iontophoretic device placed upon body 40 to illustrate the iontophoretic process. Each electrode 20 and 30 comprises an element 22 and 32 composed of an adhesive material mixed with an ionic substance 24 and 34 and a backing sheet 26 and 36. The positively charged ionic substance 24 in element 22 of electrode 20 is the drug which is to be introduced into the body. Electrodes 20 and 30 are coupled to a current source, such as 10, which includes a source of electrical pulses, which may be of a type suitable for either nerve or muscle stimulation. The current source is disposed in a housing carrying the electrodes 20 and 30 as is more fully explained with reference to Figs. 4 and 6. When a current is generated by a current source, such as 10, and applied to electrodes 20 and 30 in place upon body 40, a current will flow through body 40 in the direction shown at arrow 45. The current will cause positively charged ionic substance 24 to be driven out of electrode 20 into body 40. It should be understood that the word body is used in its most general sense, and can include plant, animal and human bodies.

Fig. 3 shows a sectional view of an alternative embodiment of the active electrode of the iontophoretic device according to the invention. In this embodiment a sheet 51 of an adhesive and ionic substance mixture is affixed to a backing sheet 54 by means of adhesive 56. The backing sheet 54 is made of a conductive material and serves as a current disperser. Adhesive 56 may be either the adhesive used in making sheet 51 or any other suitable adhesive.

An ambulatory iontophoretic device according to the invention is shown in Fig. 4. In this system the current source pulse generator is contained within housing 60 to which electrode plates 62 and 64 are directly affixed. The electrodes are applied to plates 62 and 64. In Fig. 4 an electrode 70 is shown applied to plate 62. The position the second electrode would take if it were applied to plate 64 is shown in ghost at 80A. An electrode such as may be applied to plate 64 of the iontophoretic current generator of Fig. 4 is shown in Fig. 5a as it may be manufactured and sold. The electrode element 80, has its broad surfaces covered by protective release liners 82 and 84. The release liners 82 and 84 are preferably of polyethylene coated or wax impregnated paper, but may also be formed of plastic, cloth, fiber, or any other suitable material possessing release characteristics. Fig. 5b shows the electrode of Fig. 5a with the upper protective liner 84 removed in preparation for applying electrode element 80 to a plate such as 64 of the iontophoretic current generator of Fig. 4. It may be appreciated that the invention makes compact, ambulatory iontophoretic current generators such as shown in Fig. 4 much more practical because of the simplicity of the electrodes.

Fig. 6 shows the bottom view of the iontophoretic current generator of Fig. 4 having one electrode 70 attached. This view shows the square shape of the electrode in this embodiment. Obviously, many other shapes and sizes of electrodes could be used. It will be appreciated that the electrodes of Fig. 5a could be manufactured in preformed shapes or very large sheets, in which case individual electrodes of a shape and size suitable for application to a current generator could be cut out of the large sheet as necessary. Or alternatively, the large sheet could be manufactured with partial pre-cuts or grooves along the sheet defining the boundaries of individual electrodes, so that the individual electrodes could be detached from the sheet along the cuts or grooves.

One of the features of the invention is the fact that in the embodiment shown in Figs. 4 and 6, electrodes 70 and 80 serve not only as a means of holding the electrodes to the body, but also as a means for holding the iontophoretic device to the body. In the embodiment shown, safety strap 90 is provided as an auxiliary means for holding the device to the body due to the substantial mass of the current generator.

The electrode elements such as 22, 51 and 70 constitute the means for holding the ionic substance in such a manner that it is available for being driven into the body by the current.

In the embodiments shown, electrode elements 22, 51 and 70 are the active electrodes and the ionic substance 24, 52 and 73 respectively is positively charged and comprises the substance which it is desired to introduce into the body. In this embodiment electrodes 32 and 80 are the indifferent electrodes and the ionic substance 34 and 83 may have either positive or negative charge, or both. In some embodiments it may be desirable to drive a negatively charged ionic substance into the body, in which case the negative electrode would be active and the positive electrode would be the ground. Further, in some embodiments, for example embodiments in which the electrode elements such as 30 and 80 are applied to an iontophoretic generator in which the polarity of the electrode plates such as 62 and 64 is reversible or embodiments in which simultaneous delivery of both positive and negative drugs is desirable, the ionic substance 34 and 83 may also be a substance which it is desired to introduce into the body. In some instances it may be desired to introduce more than one substance of the same charge into the body and thus several ionic substances may be included in the same active electrode element.

The composition of the electrode elements such as 22, 32, 51, 70 and 80 may include ingredients to control or alter the physical properties of the element. Tackifiers may be added to control the tackiness, humectants and water may be added to control the wetness, preservatives may be added to extend the shelf life and/or the useful life of the product, or inert fillers may be added to control the bulk or dilute or adjust other properties. Preferably the physical properties are adjusted so that the elements 22, 32, 51, 70 and 80 are solid, that is, their consistency is such that the material does not perceptively flow. It is also contemplated that the composition may be manufactured and sold in a liquid form which upon application to the plates such as 62 or backing materials such as 26, 36 and 54 changes to a tacky liquid or solid form by drying, chemical reaction or otherwise.

Ingredients may also be added to the composition to color it. The coloring of the electrode element may be used as a code to identify the ionic substance which is admixed in the particular electrode or electrode element.

The iontophoretic electrode composition used in the device of this invention consists essentially of an adhesive material in admixture with an ionic substance. The preferred adhesive contains one or more synthetic or naturally occurring polar, non-ionic polymers, a tackifier, a humectant and water.

As stated above, the adhesive materials contain essentially non-ionic, polar, synthetic and/or naturally occurring polymeric compounds. The non-ionic nature is preferable so that the adhesive does not interfere with the iontophoretic process. The polar nature is preferable since it ensures that the ionic drug will be soluble in the adhesive material. Essentially non-ionic, polar synthetic polymers suitable for use in the adhesive material of the iontophoretic electrodes of this invention are exemplified by the following materials: poly(acrylamide), poly(2-hydroxyethyl acrylate), poly(2-hydroxypropyl acrylate), poly(n-vinyl-2-pyrrolidone), poly(n-methylol acrylamide), poly(diacetone acrylamide), poly(2-hydroxyethyl methacrylate), poly(2-hydroxy propyl methacrylate), poly(vinyl alcohol), poly(ethylene oxide), poly(propylene oxide), and poly(allyl alcohol). Hydroxyl functional condensation polymers (i.e. polyesters, polycarbonates, polyurethanes) are also examples of essentially non-ionic, polar synthetic polymers suitable for use in the adhesive material of the iontophoretic electrodes of the invention: Essentially non-ionic, polar naturally occurring polymers (or derivatives thereof) suitable for use in the adhesive material of the iontophoretic electrodes of this invention are exemplified by the following materials: cellulose ethers, methyl cellulose ethers, cellulose and hydroxylated cellulose, methyl cellulose and hydroxylated methyl cellulose, gums such as guar, locust, karaya, xanthan and gelatin.

Tackifiers which may be included in the adhesive composition are exemplified by the following materials; polybutene, terpene resins, rosin resins, paraffinic oils, glycols glycerine, and sorbitol. Humectants which may be included are exemplified by: glycols, glycerine and sorbitol.

A variety of ionic substances intended to be introduced into the body may be intermixed with the adhesive. Some of the ionic substances are pilocarpine nitrate, lidocaine hydrochloride, hydrocortisone derivatives, acetic acid, fluoride, penicillin and dexamethasone sodium phosphate.

The electrodes formed using these ionic substances are generally used as the active electrodes, although it would be possible to use them also for the indifferent electrodes in certain circumstances.

Other ionic substances which may be mixed with the adhesive are salts such as potassium sulfate or sodium chloride. The electrodes formed using these ionic substances generally would be useful only for the indifferent electrodes.

The following examples are illustrative of the processes and materials used to obtain the electrode compositions used in the device of the invention.

Example I

11.3 grams of pilocarpine nitrate were dissolved in 270 ml of deionized water in a 1 liter flask. To the former solution 300 grams of glycerine followed by 300 grams of polybutene were added. Next, a previously mixed combination of 225 grams of gelatin and 37.5 grams of polyvinyl pyrrolidone was added. The resulting layers of components were stirred until the mixture thickened and was difficult to stir (about 2—3 minutes). A water bath heated the mix for about one hour at which time the temperature was approximately 65°C, and the mix was again fluid. The fluid mix, with continued heating, was stirred five minutes or longer to insure homogenity, at which point the temperature was approximately 75°C. The composite was poured into a polyethylene plastic pan, covered with aluminum foil and refrigerated until set.

About 3 hours later the composite was weighed into approximately 100 gram amounts. These were placed within a 165×165×3.2 mm brass frame between sheet of Mylar. The sandwiched composite was placed between the platens of a compression molder at about 55°C and subjected to 89 kN ram force for 2 to 3 minutes.

The sheet of pressed composite was cut with a stainless steel scalpel into squares 28.6×28.6 mm while still between Mylar. The electrodes formed in this manner were applied to an electrode plate 62 of an iontophoretic generator such as shown in Figs. 4 and 6, as previously described, with relative ease. After the iontophoretic process described above was completed it was found that there was good uniform pilocarpine introduction into the body under the entire pad with minimal hot spots and burning.

Example II
The materials and procedures as in Example I were used except that 11.3 grams potassium sulfate was substituted for the pilocarpine nitrate, and 10 ml of 4% FD4C Blue 1 was substituted for 10 ml of the deionized water. The electrode thus formed was found to be easily applied to the electrode plate 64 of the iontophoretic current generator shown in Figs. 4 and 6 and used with good results as the indifferent electrode during the above-described iontophoretic process.

Example III
The same materials and procedures as in Example I were used, except that the pilocarpine nitrate was omitted. After the composite had solidified, 98 g of the composite was cut, remelted and mixed with 10 ml of 20% lidocaine solution (2% drug by weight). After resolidification this was made into pads and used in the iontophoretic process with good results as in Example I.

The various components, including the adhesive and ionic substance given in the examples, and any other components which may be used, are preferably provided in such relative amounts as to form a flexible, self-supporting material with substantial shape retention, which is adhesive and which is electrically conductive. The components may be adjusted to form a composition of other physical consistency, as discussed above, if desired.

In terms of the percentage of weight of the total adhesive composition it has been found that the following weight percentages of the following components given in the examples provides electrodes of desirable adhesiveness and physical consistency:

| Component | Percent of weight |
|---|---|
| polyvinyl pyrrolidone | 2% to 7% |
| gelatin | 15% to 35% |
| glycerin | 20% to 35% |
| polybutene | 15% to 30% |
| water | 20% to 35% |

Further, it has been found that polybutene having a molecular weight between 1000 and 3000 is preferable.

The composition with pilocarpine nitrate may be used in the diagnosis of cystic fibrosis. For this purpose the iontophoretic device having an electrode comprising the mixture of pilocarpine nitrate and an adhesive material is applied to the body, and electrical current is driven through the mixture to drive the pilocarpine nitrate into the body. The body is then allowed to sweat and the sweat is analyzed for its chloride or sodium content to determine the presence of cystic fibrosis, as disclosed in the prior art. The device according to the invention permits precise control of the pilocarpine nitrate introduced into the body. At the same time it substantially simplifies the medical procedures necessary to introduce the pilocarpine nitrate, thus making it much more practical to use the diagnostic method as a means of screening large numbers of persons, such as all newborn infants, for cystic fibrosis. The more controlled and easier application also results in the iontophoretic method being much more practical with the other ionic substances listed above. It will be appreciated that the same advantage, and other advantages may be obtained with devices having electrodes including any ionic substances for which the iontophoretic process has been shown, or will be shown to be applicable.

**Claims**

1. An iontophoretic device for iontophoretically introducing an ionic drug (73, 83) into body tissue, through the skin, said device comprising a housing (60) for a pulse generator having affixed thereto at least one electrode plate (62, 64) which has removably applied thereto an adhesive ionto-phoretic electrode sheet (70, 80A) composed of a mixture of an adhesive and said ionic drug (73, 83), one of the broad surfaces of said adhesive sheet (70, 80A) electrically coupling the electrode sheet (70, 80A) to the electrode plate (62, 64) and the other of the broad surfaces of said adhesive sheet (70, 80A) serving as means for coupling the adhesive electrode sheet and thereby the housing (60) to the skin.

2. An iontophoretic electrode sheet for use in a device as claimed in claim 1, wherein said adhesive is a polar, non-ionic adhesive.

3. An iontophoretic electrode sheet as claimed in claim 2, wherein said ionic drug (73, 83) is pilocarpine nitrate, lidocaine hydrochloride, hydrocortisone derivatives, acetic acid, fluoride, penicillin, or dexamethasone sodium phosphate.

4. An iontophoretic electrode sheet as claimed in claim 2 or 3, wherein said adhesive comprises a tackifier, water and one or more components selected from the group of synthetic or naturally occurring polar, non-ionic polymers.

5. An iontophoretic electrode sheet as claimed in one of claims 2 to 4 and further including a humectant.

6. An iontophoretic electrode sheet as claimed in one of claims 2 to 5 and further including a thickening agent.

7. An iontophoretic electrode sheet as claimed in one of claims 4 to 6, wherein said one or more polymers includes polyvinyl pyrrolidone and said polyvinyl pyrrolidone comprises between two percent and seven percent of the total weight of said adhesive.

8. An iontophoretic electrode sheet as claimed in one of claims 4 to 7, wherein said one or more polymers includes gelatin and said gelatin comprises between fifteen percent and thirty percent of the total weight of said adhesive.

9. An iontophoretic electrode sheet as claimed in one of claims 4 to 8, wherein said tackifier is polybutene having a molecular weight between 1000 and 3000 and said polybutene comprises between fifteen percent and thirty percent of the total weight of said adhesive.

10. An iontophoretic electrode sheet as claimed in one of claims 4 to 9, wherein the water comprises between twenty percent and thirty-five percent of the total weight of said adhesive.

11. An iontophoretic electrode sheet as claimed in one of claims 5 to 10, wherein said humectant includes glycerine and said glycerine comprises between twenty percent and thirty-five percent of the total weight of said adhesive.

## Patentansprüche

1. Iontophoretisches Gerät zum iontophoretischen Einbringen eines ionischen Arzneimittels (73, 83) in Körpergewebe über die Haut, mit einem Gehäuse (60) für einen Impulsgenerator, an dem mindestens eine Elektrodenplatte (62, 64) befestigt ist, auf die eine iontophoretische Elektrodenhaftschicht (70, 80A) aus einem Gemisch eines Klebers und des ionischen Arnzeimittels (73, 83) lösbar aufgebracht ist, wobei eine der Breitseiten der Haftschicht (70, 80A) die Elektrodenschicht (70, 80A) mit der Elektrodenplatte (62, 64) elektrisch koppelt und die andere Breitseite der Haftschicht (70, 80A) als Mittel zum Koppeln der Elektrodenhaftschicht und damit des Gehäuses (60) mit der Haut dient.

2. Iontophoretische Elektrodenschicht zur Verwendung in einem Gerät nach Anspruch 1, wobei der Kleber ein polarer, nichtionischer Kleber ist.

3. Iontophoretische Elektrodenschicht nach Anspruch 2, wobei das ionische Arzneimittel (73, 83) Pilokarpinnitrat, Lidocainhydrochlorid, ein Hydrokortisonderivat, Essigsäure, Fluorid, Penizillin oder Dexamethasonnatriumphosphat ist.

4. Iontophoretische Elektrodenschicht nach Anspruch 2 oder 3, wobei der Kleber einen Klebrigmacher, Wasser und eine oder mehrere Komponenten enthält, die aus der aus synthetischen oder natürlich vorkommenden polaren, nichtionischen Polymeren bestehenden Gruppe ausgewählt sind.

5. Iontophoretische Elektrodenschicht nach einem der Ansprüche 2 bis 4, die ferner ein Feuchthaltemittel aufweist.

6. Iontophoretische Elektrodenschicht nach einem der Ansprüche 2 bis 5, die ferner ein Eindickmittel aufweist.

7. Iontophoretische Elektrodenschicht nach einem der Ansprüche 4 bis 6, wobei zu dem einen oder den mehreren Polymeren Polyvinylpyrrolidon gehört und das Polyvinylpyrrolidon zwischen zwei Prozent und sieben Prozent des Gesamtgewichts des Klebers ausmacht.

8. Iontophoretische Elektrodenschicht nach einem der Ansprüche 4 bis 7, wobei zu dem einen oder den mehreren Polymeren Gelatine gehört und die Gelatine zwischen fünfzehn Prozent und dreißig Prozent des Gesamtgewichts des Klebers ausmacht.

9. Iontophoretische Elektrodenschicht nach einem der Ansprüche 4 bis 8, wobei der Klebrigmacher Polybuten mit einem Molekulargewicht zwischen 1000 und 3000 ist und das Polybuten zwischen fünfzehn Prozent und dreißig Prozent des Gesamtgewichts des Klebers ausmacht.

10. Iontophoretische Elektrodenschicht nach einem der Ansprüche 4 bis 9, wobei das Wasser zwischen zwanzig Prozent und fünfunddreißig Prozent des Gesamtgewichts des Klebers ausmacht.

11. Iontophoretische Elektrodenschicht nach einem der Ansprüche 5 bis 10, wobei das Feuchthaltemittel Glyzerin aufweist und das Glyzerin zwischen zwanzig Prozent und fünfunddreißig Prozent des Gesamtgewichts des Klebers ausmacht.

## Revendications

1. Dispositif d'ionophorèse pour l'introduction par ionophorèse d'un médicament ionique (73, 83) dans le tissu du corps, à travers la peau, ce dispositif comprenant un boitier (60) pour un générateur d'impulsions sur lequel est fixée au moins une plaque-électrode (62, 64) sur laquelle est appliquée de façon amovible une feuille-électrode adhésive d'ionophorèse (70, 80A) composée d'un mélange d'adhésif et dudit médicament ionique (73, 83), l'une des grandes surfaces de ladite feuille adhésive (70, 80A) couplant électriquement la feuille-électrode (70, 80A) à la plaque-électrode (62, 64) et l'autre des grandes surfaces de ladite feuille adhésive (70, 80A) servant de moyen pour coupler la feuille-électrode adhésive et ainsi le boitier (60) avec la peau.

2. Feuille-électrode d'ionophorèse destinée à être utilisée dans un dispositif tel que défini dans la revendication 1, dans laquelle ledit adhésif est un adhésif polaire non ionique.

3. Feuille-électrode d'ionophorèse suivant la revendication 2, dans laquelle ledit médicament ionique (73, 83) est le nitrate de pilocarpine, le chlorhydrate de lidocaine, les dérivés de l'hydrocortisone, l'acide acétique, les florures, la pénicilline ou le phosphate sodé de déxaméthasone.

4. Feuille-électrode d'ionophorèse suivant la revendication 2 ou 3, dans laquelle ledit adhésif est constitué par un agent d'adhérence, de l'eau et un ou plusieurs composants choisis parmi le groupe des polymères non ioniques synthétiques ou naturels.

5. Feuille-électrode d'ionophorèse suivant l'une des revendications 1 à 4, comprenant en outre un humectant.

6. Feuille-électrode d'ionophorèse suivant l'une des revendications 2 à 5, comprenant en outre un agent épaississant.

7. Feuille-électrode d'ionophorèse suivant l'une des revendications 4 à 6, dans laquelle le ou lesdits polymères comprennent le polyvinyl pyrrolidone, celui-ci constituant entre deux pour cent et sept pour cent du poids total dudit adhèsif.

8. Feuille-électrode d'ionophorèse suivant l'une des revendications 4 à 7, dans laquelle le ou lesdits polymères comprennent la gélatine, celle-ci constituant entre quinze et trente pour cent du poids total dudit adhèsif.

9. Feuille-électrode d'ionophorèse suivant l'une des revendications 4 à 8, dans laquelle ledit agent d'adhèrence est un polybutène ayant un poids moléculaire compris entre 1.000 et 3.000, ce polybutène constituant entre quinze et trente pour cent du poids total dudit adhèsif.

10. Feuille-électrode d'ionophorèse suivant l'une des revendications 4 à 9, dans laquelle l'eau constitue entre vingt et trente pour cent du poids total dudit adhèsif.

11. Feuille-électrode d'ionophorèse suivant l'une des revendications 5 à 10, dans laquelle ledit humectant comprend la glycèrine, celle-ci constituant entre vingt et trente pour cent du poids total dudit adhèsif.

FIG. 1

FIG. 2

FIG. 3

FIG. 4

FIG. 5a

FIG. 5b

FIG. 6